# EUROPÄISCHE PATENTSCHRIFT

(11) **EP 1 142 544 B1**
(45) Veröffentlichungstag und Bekanntmachung des Hinweises auf die Patenterteilung: **26.03.2008**
(21) Anmeldenummer: 00107331.1
(22) Anmeldetag: 04.04.2000
(51) Int. Cl.: A61F 2/44

(54) **Zwischenwirbelkunststoffimplantat**
Intervertebral implant
Implant intervertébral

(43) Veröffentlichungstag der Anmeldung: 10.10.2001
(73) Patentinhaber: Link Spine Group, Inc., Branford, CT 06405 (US)
(72) Erfinder: Keller, Arnold, 23863 Kayhude (DE)
(74) Vertreter: Glawe, Delfs, Moll

(56) Entgegenhaltungen:
- EP-A- 0 392 076
- WO-A-99/62439
- FR-A- 2 703 580
- US-A- 4 759 766
- US-A- 5 571 189

## Beschreibung

Die Erfindung betrifft Kunststoffimplantate nach dem Oberbegriff des Anspruchs 1.

Kunststoffimplantate sind in Röntgenabbildungen aufgrund ihres mangelnden Kontrastunterschiedes zum Körpergewebe nicht deutlich abbildbar. Es ist bekannt, zur Überprüfung der Position eines eingesetzten Kunststoffimplantats dieses mit einem Metalldraht zu versehen, welcher in Röntgenaufnahmen sichtbar ist (GB 1477149, GB 1409051, US 4892548). Zu diesem Zweck kann in die Oberfläche des Implantats eine umlaufende Nut eingearbeitet sein, in die der Röntgenkontrastdraht eingelegt wird. Zur Befestigung des Drahtes am Implantat werden gewöhnlich die beiden Drahtenden miteinander verzwirnt. Bei der Benutzung des Kunststoffimplantats kann es zu einer Belastung und zum Bruch des Drahtes kommen. Falls der Draht dann aus seiner vorgesehenen Position auswandert, kann es erforderlich werden, ihn durch einen Eingriff zu entfernen.

Um der Gefahr eines Drahtbruchs entgegenzuwirken, ist es bekannt, die Enden des Metalldrahtes nicht miteinander zu verzwirnen, sondern abzuknicken und in zwei senkrecht in die Oberfläche eingearbeitete Bohrungen einzustecken. Es wurde auch versucht, das eine Drahtende mit einer Öse zu versehen und das andere Ende so darin festzuhaken, daß ein gewisses Spiel verbleibt. Die Ergebnisse dieser Versuche waren jedoch nicht zufriedenstellend.

Aufgabe der Erfindung ist es daher, ein Kunststoffimplantat der beschriebenen Art zu schaffen, bei dem die Gefahr des Bruchs und/oder die Gefahr des Auswanderns des Röntgenkontrastdrahtes vermieden ist.

Die Erfindung liegt in den kennzeichnenden Merkmalen des Anspruchs 1, nämlich darin, daß der den Röntgenkontrastdraht aufnehmende Kanal nach außen ganz oder teilweise geschlossen ist, um den Draht darin zu sichern, und daß eine Öffnung zum Einbringen des Drahts in den Kanal vorgesehen ist.

Dadurch wird der Draht im Kanal gehalten und ein Auswandern desselben nach außen verhindert. Geschlossen ist jede räumliche Gestaltung, durch welche ein Auswandern eines im Kanal gehaltenen Drahtes nach außen unterbunden wird. Auf die Verbindung der Drahtenden miteinander kann verzichtet werden. Der Draht bildet dann eine offene Schleife, die sich nachgiebig gegenüber Verformungen des Implantats verhält und keinen nennenswerten Belastungen ausgesetzt ist. Beispielsweise kann bei einer Ausdehnung des Implantats durch ein Gleiten des Drahtes längs des Kanals eine Anpassung des Drahts an die vergrößerte Länge des Implantatumfangs erfolgen. Dies gilt sowohl bei Verwendung einer offenen Drahtschleife als auch im Falle eines Bruchs einer geschlossenen Drahtschleife.

Die Angabe "nach außen" ist auf die Querschnittsform des Kanals bezogen. Der Verlauf des Kanals ist nicht auf bestimmte Formen begrenzt. Er folgt in der Regel dem Verlauf des Randes. Der Kanal muß nicht in sich geschlossen sein.

Der Kanal kann im wesentlichen durchgängig über seine gesamte Länge oder den größten Teil seines Verlaufs geschlossen sein. Es ist jedoch im allgemeinen nicht schädlich, wenn diese Schließung in einzelnen Abschnitten des Kanals fehlt, solange nur die hinreichend sichere Halterung des Drahtes darin gewährleistet ist. Es können sogar lediglich einige über den Umfang verteilte, geschlossene Stellen ausreichen.

Die Schließung des Kanals nach außen muß im Querschnitt nicht vollständig sein; umfaßt sind vielmehr auch teilweise geschlossene Kanäle. Im Hinblick auf die Herstellbarkeit durch spangebende Formung wird eine nur teilweise Schließung der Kanäle nach außen bevorzugt.

Die teilweise Schließung des Kanals wird vorzugsweise durch eine Verengung seines Profils nach außen bewirkt, deren Weite geringer als die Drahtdicke ist. Damit der Draht eindeutig positioniert ist, ist sein Durchmesser zweckmäßigerweise nur wenig geringer als der Kanaldurchmesser. Der Draht kann beispielsweise durch Eindrücken durch die Verengung von außen in den Kanal eingebracht werden. Zu diesem Zweck ist das Implantat bei einer möglichen Ausführungsform im Bereich der Verengung so elastisch und ist zudem die Weite der Verengung so groß bemessen, daß die Verengung zur Einbringung des Drahtes elastisch aufgeweitet werden kann.

Bevorzugt wird jedoch eine andere Ausführungsform, bei der der Kanal auf einer zum Einführen des Drahts ausreichenden Umfangslänge eine Einschuböffnung aufweist.Einerseits muß die Einschuböffnung eine hinreichende Länge (in Längsrichtung des Kanals) aufweisen, um ein bequemes Einschieben des Drahts in den Kanal zu ermöglichen. Andererseits darf die Einschuböffnung nicht zu lang sein, damit der Austritt des Drahts im Falle von Längsbewegungen des Drahts im Kanal verhindert wird. In dieser Hinsicht ist es vorteilhaft, wenn beide Kanalenden in der Einschuböffnung münden, und zwar fluchtend miteinander, so daß ein aus einer Kanalöffnung auswanderndes Drahtende auf der gegenüberliegenden Seite der Einschuböffnung in die andere Kanalmündung wieder eintritt. So bleibt der Draht innerhalb des Kanals gefangen. Wenn der Kanal gekrümmt ist, ist es zweckmäßig, wenn der Draht im entspannten, unmontierten Zustand etwa dieselbe Krümmung wie der Kanal besitzt. Falls sein Ende aus einer Kanalmündung auswandert, so folgt er der Fortsetzung dieses gekrümmten Wegs, auf dem auch die andere Kanalmündung liegt, so daß er um so leichter in diese andere Kanalmündung wieder eintritt. Der Begriff "fluchtend" ist in diesem Zusammenhang so zu verstehen, daß ihre gekrümmt verlaufenden Achsen übereinstimmen.

Eine hinreichende Sicherung der Drahtenden in der Entnahmeöffnung verlangt aber nicht unbedingt, daß ein aus einer Kanalmündung auswanderndes Drahtende in die andere Kanalmündung wieder eintritt; es genügt, wenn ein ggf. auswanderndes Drahtende an einer gegenüberliegenden Wand der Entnahmeöffnung anschlägt und dadurch an weiterer, den Draht aus den Kanal herausführender Bewegung gehindert wird.

Ferner kann ein Auswandern des Drahtes aus der Einschuböffnung dadurch verhindert werden, daß die Drahtenden, die in ihrer montierten Position jeweils aus den Kanalmündungen heraus vorstehen, von der Kanalachse weggebogen oder abgeknickt werden. Einer Verschiebung des Drahtes sind dann durch den Anschlag der abgebogenen bzw. abgeknickten Enden an den zugeordneten Kanalenden oder anderen durch die Einschuböffnung gebildeten Wänden Grenzen gesetzt. Um Raum für die abgebogenen oder abgeknickten Enden zu schaffen, ist die Einschuböffnung zweckmäßigerweise gegenüber dem Kanalquerschnitt erweitert.

Das Abbiegen oder -knicken geschieht vorzugsweise nicht unmittelbar an den Kanalenden, sondern in einem gewissen Abstand davon, wodurch ein gewisses Spiel zum Ausgleich der oben beschriebenen Längenänderungen verbleibt.

Um beim Einschieben die Reibung zwischen dem Draht und der äußeren Kanalwand zu begrenzen, ist es vorteilhaft, wenn der umlaufende Kanal monoton gekrümmt ist. Stellen mit einer starken Krümmung werden vorteilhafterweise vermieden. Damit sich der Draht beim Einschieben in den Kanal nicht in der Verengung verklemmen kann, beträgt die Verengungsweite höchstens 80 %, vorzugsweise höchstens 70 % und weiter vorzugsweise höchstens 60 % des Drahtdurchmessers. Vorteilhafterweise ist der in den Kanal einzuschiebende Draht mindestens an seinem voranzuführenden Ende abgerundet, damit er sich leichter einschieben läßt.

Besonders gewinnbringend ist die vorliegende Erfindung für die Verwendung bei Zwischenwirbel-Endoprothesen mit einem Gleitkern aus nachgiebigem Kunststoff wie Polyethylen, bei dem die Möglichkeit besteht, daß sich der Gleitkern durch Druckbelastung unter Vergrößerung seines Umfangs ausdehnt.

Die Erfindung wird im folgenden anhand eines vorteilhaften Ausführungsbeispiels mit Bezug auf die beigefügten Zeichnungen erläutert. Es zeigen:

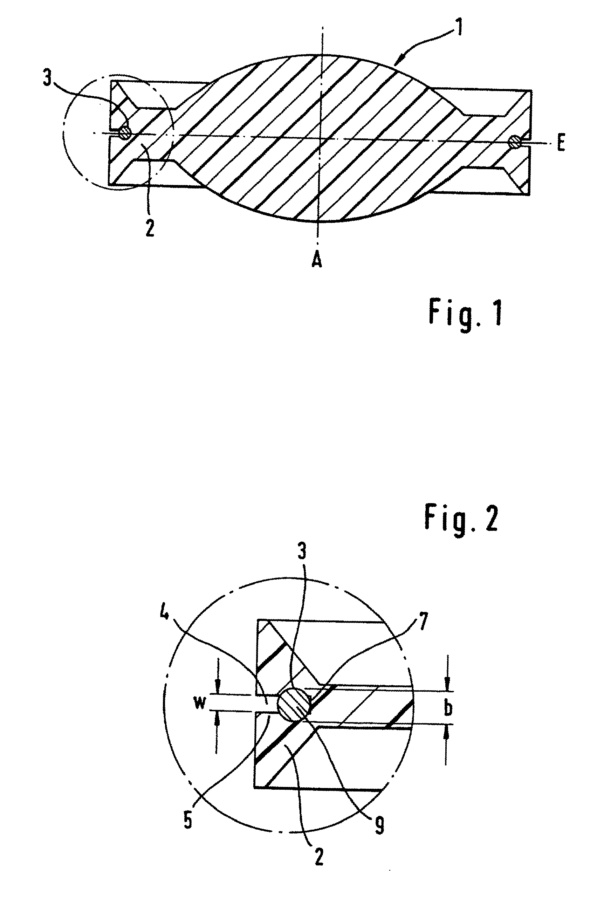

- Fig. 1: einen vertikalen Querschnitt durch den Gleitkern einer Zwischenwirbel-Endoprothese;
- Fig. 2: einen vergrößerten Ausschnitt aus Fig. 1 im Bereich des Kanals für den Röntgenkontrastdraht;
- Fig. 3: eine Draufsicht auf den Gleitkern aus Fig. 1 mit einem horizontalen Teilschnitt im Bereich der Einschuböffnung für den Draht; und
- Fig. 4: eine Seitenansicht auf den Gleitkern aus den Fig. 1, 2 im Bereich der Einschuböffnung.

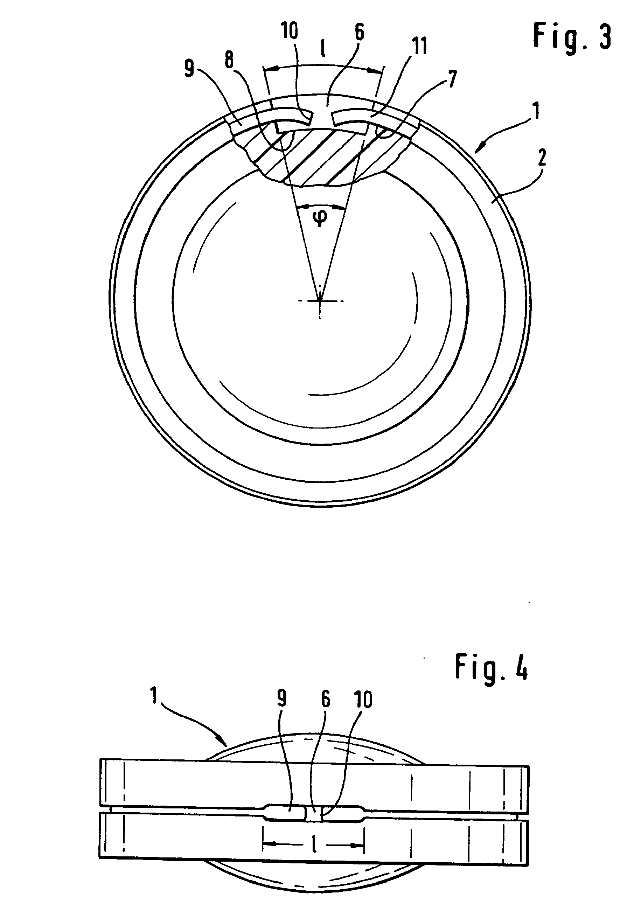

Der Gleitkern 1 ist oben und unten von zwei nicht gezeigten Anschlußplatten eingefaßt; die so gebildete Zwischenwirbel-Endoprothese ist zwischen zwei Wirbelkörper einzusetzen. Der Gleitkern 1 ist im wesentlichen diskusförmig mit einem ringförmig vorspringenden Rand 2. Darin ist eine umlaufende Nut 3, 4 zur Aufnahme eines Röntgenkontrastdrahtes 9 vorgesehen. Wie aus Fig. 3 ersichtlich, folgt der Verlauf der Nut 3, 4 im wesentlichen dem kreisförmigen Rand des Gleitkerns. Dieser besteht aus nachgiebigem Kunststoff, insbesondere Polyethylen. Ein in die Nut 3, 4 eingebrachter Metalldraht 9 ist auf Röntgenabbildungen sichtbar und erlaubt eine Positionskontrolle des Gleitkerns 1 sowie die Beurteilung der Artikulation mit dem nicht gezeigten Gegenlager.

Die Nut 3, 4 besteht aus einem innen liegenden Kanal 3 und einer den Kanal nach außen hin begrenzenden Profilverengung 4. Der Kanal 3 wird durch die Verengung 4 nach außen teilweise geschlossen. Die Verengung 4 wird von einem parallel zum Kanal 3 verlaufenden Schlitz in der äußeren Kanalwand 5 gebildet. Die Schlitzweite beträgt beispielsweise 0.4 mm, während die Kanalbreite b = 0.75 mm beträgt. Die Schlitzweite w im Verhältnis zur Kanalbreite b beträgt damit ungefähr 50 %.

Im allgemeinen liegt dieser Wert im Bereich von 20 bis 80 %, vorzugsweise 30 bis 70 %, weiter vorzugsweise 40 bis 60 %. Der Schlitz dient bei diesem Ausführungsbeispiel dazu, die spangebende Formung des Kanals 3 zu ermöglichen. Der Drahtdurchmesser beträgt 0.7 mm und ist damit etwas geringer als der Kanaldurchmesser und größer als die Schlitzweite.

Der Kanal 3 weist in einem Bereich des Azimutwinkels ϕ von vorzugsweise 20 bis 40°, im vorliegenden Beispiel 30°, eine Einschuböffnung 6 auf mit einer Umfangslänge 1 von 10 mm. Sie wird durch eine Ausschneidung im Rand 2 der Prothese erzeugt. Der die Verengung 4 bildende Schlitz ist im Bereich der Einschuböffnung auf die Kanalweite oder wenig mehr erweitert. Wie aus Fig. 3 ersichtlich, ist sie über den Kanalboden 7 hinaus tiefer eingeschnitten, so daß der Boden 8 der Einschuböffnung 6 radial weiter innen liegt.

Die Länge des mit dem Gleitkern 1 verwendeten Röntgenkontrastdrahts 9 ist wenig geringer als die Umfangslänge des Kanals 3. Der Draht wird vor seiner Einbringung in den Kanal 3 kreisbogenförmig etwa entsprechend dem Verlauf des Kanals 3 gebogen. Die Drahtenden 10 liegen in der Einschuböffnung frei. Ein gegenseitiger Abstand der Drahtenden von einigen mm ist dabei erwünscht. Bei der Montage wird er in den Kanal 3 durch die Einschuböffnung 6 eingeschoben, bis er seine in den Fig. 3 und 4 gezeigte, vorgesehene Position erreicht hat. Dies wird durch die Abrundung oder Anfasung der Kanten an seiner vorangehenden Stelle erreicht. Im Anschluß an den Einschubvorgang werden die in der Einschuböffnung 6 positionierten Enden 10 des Metalldrahts 9 nach innen eingebogen, wie aus Fig. 3 ersichtlich. Die Abbiegung ist so gering oder erfolgt in einem solchen Abstand von der Kanalmündung 11, daß ein entsprechendes Spiel für den nachfolgend geschilderten Längenausgleich verbleibt. Ein Auswandern des Drahtes 9 aus der Einschuböffnung 6 wird durch das Anschlagen der abgebogenen Drahtenden 10 an der jeweils zugeordneten Kanalmündung oder den jeweils gegenüberliegenden Wänden der Einschuböffnung 6 verhindert. Aufgrund des Überstandes der Drahtenden 10 über die Kanalmündung 11 können diese erfaßt und der Draht ggf. leicht wieder entfernt bzw. ausgetauscht werden.

Um den Draht im Implantat zu sichern und ein Auswandern seiner Enden 10 aus der Einschuböffnung 6 zu verhindern, ist es nicht unbedingt erforderlich, die Drahtenden 10 abzubiegen oder abzuknicken. Wenn sie in ihrer ursprünglich geraden oder entsprechend dem Kanal gekrümmten Form verbleiben, hindert die Form der Einschuböffnung sie daran, das Implantat zu verlassen. Wandert ein Drahtende 10 aus der zugehörigen Kanalmündung 11 heraus, so tritt es entweder in die gegenüberliegende Kanalmündung 11 wieder ein, wenn diese Öffnungen entlang der Kanalachse miteinander fluchten, oder dieses Drahtende 10 stößt gegen die Wand der Einschuböffnung, die die gegenüberliegende Kanalmündung 11 umgibt.

Eine Druckbelastung auf den Gleitkern 1 entlang der Prothesenachse A wird aufgrund der Nachgiebigkeit des Materials in eine Ausdehnung in der zur Achse A lotrechten Mittelebene E umgesetzt. Dies führt zu einer Vergrößerung der Umfangslänge des Gleitkerns 1. In der dargestellten Ausführungsform wird ein entsprechender Längenausgleich der offenen Drahtschleife dadurch bewirkt, daß der Draht 9 an einem oder beiden Enden 10 um ein entsprechendes Stück in den Kanal 3 hineingezogen wird. Bei der Entspannung des Gleitkerns 1 werden die Drahtenden 10 dementsprechend wieder etwas herausgeschoben.

## Patentansprüche

1. Kunststoffimplantat mit einem umlaufenden Kanal (3), der einen Röntgenkontrastdraht (9) enthält, **dadurch gekennzeichnet, daß** der Kanal (3) bis auf eine Öffnung zum Einbringen des Drahts (9) zur Sicherung des Drahts (9) im Kanal (3) nach außen ganz oder teilweise geschlossen ist.

2. Kunststoffimplantat nach Anspruch 1, **dadurch gekennzeichnet, daß** der Kanal (3) durch eine Verengung (4) seines Profils, deren Weite geringer als der Durchmesser des Drahtes (9) ist, nach außen teilweise geschlossen ist.

3. Kunststoffimplantat nach Anspruch 1 oder 2, **dadurch gekennzeichnet, daß** der Kanal (3) mit einer zum Einschieben des Drahtes (9) geeigneten Einschuböffnung (6) versehen ist.

4. Kunststoffimplantat nach einem der Ansprüche 1 bis 3, **dadurch gekennzeichnet, daß** der Kanal (3) monoton gekrümmt verläuft.

5. Kunststoffimplantat nach einem der Ansprüche 1 bis 4, **dadurch gekennzeichnet, daß** die Kanten des Drahts (9) mindestens an einem Ende abgerundet oder angefast sind.

6. Kunststoffimplantat nach einem der Ansprüche 4 bis 5, **dadurch gekennzeichnet, daß** die Kanalmündungen (11) in der Einschuböffnung (6) miteinander fluchten.

7. Kunststoffimplantat nach einem der Ansprüche 4 bis 5, **dadurch gekennzeichnet, daß** die Einschuböffnung (6) auf ihrer einer Kanalmündung (11) gegenüberliegenden Seite eine Anschlagwand für das aus dieser Kanalmündung (11) austretende Drahtende (10) aufweist.

8. Kunststoffimplantat nach einem der Ansprüche 4 bis 7, **dadurch gekennzeichnet, daß** die Einschuböffnung (6) gegenüber dem Kanal erweitert ist und wenigstens ein Ende (10) des Drahts (9) darin abgebogen ist.

## Claims

1. Plastic implant with a circumferential channel (3) for receiving a radiographic contrast wire (9), **characterized in that**, in order to secure the wire (9) in the channel (3), the channel (3) is completely or partially closed off from the outside, except for an opening for introduction of the wire (9).

2. Plastic implant according to Claim 1, **characterized in that** the channel (3) is partially closed off from the outside by means of a restriction (4) of its profile, the width of which restriction (4) is less than the diameter of the wire (9).

3. Plastic implant according to Claim 1 or 2, **characterized in that** the channel (3) is provided with an insertion opening (6) into which the wire (9) can be pushed.

4. Plastic implant according to one of Claims 1 to 3, **characterized in that** the channel (3) has a monotone curvature.

5. Plastic implant according to one of Claims 1 to 4, **characterized in that** the edges of the wire (9) are rounded or bevelled at least at one end.

6. Plastic implant according to one of Claims 4 and 5, **characterized in that** the channel orifices (11) are in alignment with each other in the insertion opening (6).

7. Plastic implant according to one of Claims 4 and 5, **characterized in that** the insertion opening (6), on its side lying opposite a channel orifice (11), has a contact wall for the wire end (10) emerging from this channel orifice (11).

8. Plastic implant according to one of Claims 4 to 7, **characterized in that** the insertion opening (6) is widened relative to the channel, and at least one end (10) of the wire (9) is bent off therein.

## Revendications

1. Implant en matière synthétique doté d'un canal périphérique (3) qui contient un fil (9) de contraste radiographique, **caractérisé en ce qu'**à l'exception d'une ouverture d'insertion du fil (9), le canal (3) est fermé totalement ou partiellement vis-à-vis de l'extérieur pour immobiliser le fil (9) dans le canal (3).

2. Implant en matière synthétique selon la revendication 1, **caractérisé en ce que** le canal (3) est partiellement fermé vers l'extérieur par un rétrécissement (4) de son profil dont la largeur est plus petite que le diamètre du fil (9).

3. Implant en matière synthétique selon les revendications 1 ou 2, **caractérisé en ce que** le canal (3) est doté d'une ouverture d'insertion (6) qui permet d'insérer le fil (9).

4. Implant en matière synthétique selon l'une des revendications 1 à 3, **caractérisé en ce que** le canal (3) présente une courbure monotone.

5. Implant en matière synthétique selon l'une des revendications 1 à 4, **caractérisé en ce que** les bords du fil (9) sont arrondis ou chanfreinés à au moins une des extrémités du fil.

6. Implant en matière synthétique selon l'une des revendications 4 à 5, **caractérisé en ce que** les embouchures (11) du canal dans l'ouverture d'insertion (6) sont alignées l'une sur l'autre.

7. Implant en matière synthétique selon l'une des revendications 4 à 5, **caractérisé en ce que** l'ouverture d'insertion (6) présente sur son côté opposé à une embouchure (11) du canal une paroi de butée pour l'extrémité (10) du fil qui sort de cette embouchure (11) du canal.

8. Implant en matière synthétique selon l'une des revendications 4 à 7, **caractérisé en ce que** l'ouverture d'insertion (6) est évasée par rapport au canal et qu'au moins une extrémité (10) du fil (9) y est repliée.
